# EUROPEAN PATENT SPECIFICATION

(11) **EP 2 795 323 B1**
(45) Date of publication and mention of the grant of the patent: **01.03.2017**
(21) Application number: 12859142.7
(22) Date of filing: 12.12.2012
(51) Int. Cl.: G01N 33/50

(54) **OPTIMIZED INSECT-BASED EX VIVO MODEL FOR TESTING BLOOD-BRAIN BARRIER PENETRATION AND METHOD FOR EXPOSING INSECT BRAIN TO CHEMICAL COMPOUNDS**
OPTIMIERTES EX-VIVO-MODELL AUF INSEKTENBASIS ZUM TESTEN DER BLUT-HIRN-SCHRANKENPENETRATION UND VERFAHREN ZUR INSEKTENGEHIRNEXPOSITION GEGENÜBER CHEMISCHEN VERBINDUNGEN
MODÈLE INSECTE EX VIVO OPTIMISÉ POUR L'ÉVALUATION DE LA PÉNÉTRATION DE LA BARRIÈRE SANG-CERVEAU ET PROCÉDÉ D'EXPOSITION D'UN CERVEAU D'INSECTE À DES COMPOSÉS CHIMIQUES

(30) Priority: 23.12.2011 DK 201101003
(43) Date of publication of application: 29.10.2014
(73) Proprietor: N2MO A/S, 2300 Copenhagen S (DK)
(72) Inventor: NIELSEN, Peter Aadal, 23840 Oxie (SE); ANDERSSON, Gunnar, 26024 Roestaanga (SE); ANDERSSON, Olga, 26024 Roestaanga (SE)
(74) Representative: Zacco Denmark A/S
(86) International application number: PCT/DK2012/050460
(87) International publication number: WO 2013/091637

(56) References cited:
- EP-A1- 2 329 265
- WO-A1-2011/018446
- WO-A1-2011/018446
- WO-A2-2007/044937
- US-B1- 7 038 106
- WILLIAMSON W. R. ET AL.: 'Preparation of Developing and Adult Drosophila Brains and Retinae for Live Imaging' JOURNAL OF VISUALIZED EXPERIMENTS 15 March 2010, page 1936., XP055157051
- NIELSEN P. A. ET AL.: 'Models for predicting blood-brain barrier permeation' DRUG DISCOVERY TODAY vol. 16, no. 11/12, June 2011, pages 472 - 475, XP055157052

## Description

### FIELD OF THE INVENTION

The present invention is directed to a method of conducting blood-brain barrier penetration studies in an insect aimed to reflect vertebrate blood-brain barrier (BBB) penetration. Investigation of BBB penetration is extremely important in drug discovery; successful CNS drugs have to cross the BBB, while BBB penetration may cause unwanted side effects for peripheral acting drugs. Specifically, the present invention relates to an optimized procedure to increase the sensitivity of the ex vivo insect models allowing the use of low exposure concentrations. The model uses insects in screening for substances with a biological effect on the brain or central nervous system and/or effect on a disease or disorder of the brain or central nervous system.

### BACKGROUND OF THE INVENTION

Pharmacologic remedy of many brain diseases is difficult because of the powerful drug exclusion properties of the blood-brain barrier (BBB). Chemical isolation of the vertebrate brain is achieved through the highly integrated, anatomically compact and functionally overlapping chemical isolation processes with the BBB in insects, also called the hemolymph-brain barrier. These include functions that need to be coordinated between tight diffusion junctions and unidirectionally acting xenobiotic transporters. Understanding of many of these processes has been hampered, because they have been experimentally difficult and expensive to disentangle in intact rodent models. Consequently, many of the processes are not well mimicked by *in vitro* or *ex vivo* BBB models.

In drug research it is extremely important to determine brain penetration both for drug candidates with CNS therapeutic potential but also for compounds which can cause CNS mediated side effects. There are in principle two strategies to measure brain penetration, a) determination of the rate of brain uptake at the initial state and b) the extent of brain exposure at the static state. The former is regarded as the most relevant because it is not compromised by metabolism, plasma binding or non specific brain binding due to the short exposure time. Because of their importance numerous methods have been developed to evaluate the rate of brain penetration. In situ brain perfusion, cell-based MDR1-MDCK assay and the PAMPA method are the most common assays in the pharmacological industry to determine BBB permeability. In situ brain perfusion is considered a golden standard method for measuring BBB permeability but in the pharmaceutical industry it does not fulfil the requirements of a method with high throughput and short-term feed back during the earliest step of drug discovery due to its labour intensity and high cost per tested candidate. For this reason the industry tend to use the high throughput but inaccurate *in vitro* models to assess BBB penetration.

In general, the *in vitro,* based, assays are regularly and routinely applied in the pharmaceutical early drug discovery phases and despite that there are major limitations by these assays most pharmaceutical companies use large batteries of *in vitro* screens. However, testing compounds in a large number of *in vitro* assays may not always reflect the *in vivo* behaviour. In fact, it is not unusual that compounds that have acceptable *in vitro* profiles turn out to have inadequate *in vivo* profiles. On the contrary, compounds may be discarded for wrong reasons.

Hence, there is a demand for intermediate models that are more reliable than *in vitro* models and at the same time faster and cheaper than traditional vertebrate *in vivo* models.

The vertebrate blood-brain barrier (BBB) represents the physiologic barrier between the brain tissue and blood vessels, which restricts the exchange of solutes and regulates absorption of exogenic agents (e.g. drugs) from the blood into the brain. The function of the central nervous system (CNS) requires a highly regulated extra-cellular environment. Anatomically the BBB in vertebrates is comprised of microvascular endothelia cells interconnected via highly specialized tight junctions (TJs), which provide a diffusion barrier and thus play a central role for permeability. Recently identified components of TJs include the claudins, a family of four-transmembrane-span proteins that are suggested to be responsible for the barrier-function of TJs (Turksen and Troy 2004). Penetration of BBB is one of the major hurdles in the development of successful CNS drugs. On the other hand, when penetration of the BBB occurs it may cause unwanted side effects for peripheral acting drugs (Schinkel 1999) (for review see Pardridge 2002).

BBB penetration is usually classified as chemistry- or biology-based. The chemistry-based penetration is linked to the lipid mediated passive diffusion, which depends on physiochemical properties of the molecule, i.e. small hydrophobic molecules tend to penetrate the BBB more readily than large and hydrophilic molecules. The biology-based penetration involves compounds that are substrates for the endogenous BBB influx or efflux transport systems, e.g. many small molecules (e.g. drugs) have shown to be substrates for the P-glycoprotein (P-gp) transporter. The P-gp's are transporter proteins located in the walls of the cells that make up the BBB (Schinkel 1999) and they are conserved among taxa as diverse as protozoa, plants, insects and mammals (Gaertner et. al. 1998). P-gp's are present in many cell-types and they play important roles in drug absorption, disposition, metabolism, and toxicity (Xia et al. 2006).

Obviously, it is crucial to have an understanding of the BBB penetration in drug discovery projects and preferably, this should be obtained without using excessive number of *in vivo* studies. Consequently, several *in vitro* absorption models are developed to predict the *in vivo* behaviour of test compounds. However, even complex *in vitro* models which include the P-gp transporter systems (Di and Kerns 2003, Summerfield et al. 2005) seem not to meet the intricate complexity of the BBB and therefore may not describe the *in vivo* behavior very well. The popular CaCo-2 model, developed to predict oral uptake, showed to be less useful for predicting brain penetration and the MDR1-MDCK assay, which is widely applied in industry, is mainly used to diagnose a Pgp efflux transport and recent studies have confirmed the low predictability of passive BBB permeability of this model (Summerfield et al., 2007). In an extensive BBB absorption study 22 compounds were tested in ten different *in vitro* BBB absorption models (Garberg 2005). None of the ten models showed correlation between *in vitro* and *in vivo* permeability. This indicates that specific BBB models not necessarily provide better prediction than non-BBB derived models. Furthermore, it was suggested that protein binding, blood-flow, metabolic stability and lipophilicity, as well as affinity for other transporters in the BBB are factors needed to be considered when predictions of *in vivo* brain distribution is to be made. Consequently, it seems as *in vitro* models are mainly suited for qualitative measurements of compounds that penetrates BBB by passive diffusion or compounds that undergo efflux via the P-gp transporter (Garberg 2005).

In vertebrates, a physically separate blood-brain barrier (BBB), primarily engineered into the single-cell layer vascular endothelium, provides an obstacle to chemical attack. At this interface, strong selective pressures have produced the integration of at least two very different cell biologic mechanisms to prevent free movement of small molecules between the humoral and CNS interstitial compartments. (Abbott, 2005; Daneman and Barres, 2005; Neuwelt et al., 2008; Zlokovic, 2008). BBB vascular endothelium cells impede the traffic of drugs by virtue of specialized lateral junction components, including tight junctions, and asymmetrically arrayed ATP binding cassette (ABC) transporters. Tight junctions prevent paracellular diffusion of charged molecules, and asymmetrically arrayed transporters actively expel lipophilic molecules back into the humoral space (Löscher and Potschka, 2005). Together, these complimentary systems prevent the majority of xenobiotics from acting on vertebrate nervous tissue (Pardridge, 2005). Although *in vivo* and *in vitro* BBB models have confirmed the importance of these two components (Schinkel et al., 1997; Nitta et al., 2003), substantial limitations hinder progress (Garberg et al., 2005). A powerful BBB model system should combine molecular genetic, genomic, chemical biology, and integrative physiology tools to probe CNS-specific chemoprotective physiology.

Recent research have shown that insects possess neural barriers that differs anatomically from the vertebrate barriers but also possess a number of highly important and relevant structures that is shared with the vertebrate barrier making the insect brain barrier a superior candidate for BBB permeability studies. Thus it has been shown that the insect barrier cells (glia) contain pleated septate and tight junctions nearly identical to the proteins that make up the vertebrate tight junctions (Wu and Beitel, 2004; Pardridge, 2005). Furthermore, it has been shown that insects possess a homology to the major ATP binding cassette (ABC) transporter (MDR/Pgp). It has also been shown that the active transporter homolog is localised at the hemolymph barrier, indicating that the subperineural glia in insects, like the vascular endothelium in vertebrates, possesses both tight junction barriers and active efflux transporters. These conclusions strengthen the utility of the insect BBB as a relevant model for screening and documentation of brain penetration in drug research. In further support of the functional relevance of the insect brain barrier model it has been shown in Drosophila that manipulation of the two principal barrier mechanisms by using measures relevant for the vertebrate barriers will open up both the diffusion and the transport barriers in Drosophila. Thus treatment of Drosophila with the known vertebrate MDR1/Pgp transport blocker Cyclosporin A (CsA) increased the ABC substrate in the Drosophila brain (Mayer et al., 2009). Thus the coincident localization of the diffusion and the xenobiotic transport barriers demonstrate that insects combine vertebrate-like drug exclusion mechanisms to maintain a chemical barrier to the brain. These observations are the base for an *ex vivo* insect model with high utility in the early screening and documentation of candidate compounds in early drug discovery phase. In contrast to other models (e.g. *in vitro* models including the PAMPA model) our model is characterised by the properties that are the bases for an appropriate function of a brain barrier and in this sense highly relevant for prediction of brain penetration in vertebrates including human. However, it is important to expose the ex vivo brain samples to low substance concentrations to avoid saturation of the Pgp transporter system.

In CNS drug discovery there is a need for efficient screening of compounds aimed at targets within the CNS system. This screening is preferentially performed in insect models with intact BBB function and will contribute to a positive selection of compounds penetrating the BBB. Such screening comprises low molecular weight compounds within a number of indications (e.g. pain, epilepsy, Parkinson, schizophrenia, Alzheimer, sleep disorders, anxiety, depression, eating disorders, drug abuse including smoking).

### SUMMARY OF THE INVENTION

The object of the present invention is to develop a new and sensitive ex-vivo insect screening model to accurately determine blood-brain barrier penetration of different chemical compounds in order to improve the compound screening procedures/processes in the early drug discovery phase. This object offers many advantages relative to prior technologies since insect models are more reliable tools for the decision-making process than the existing *in vitro and ex vivo* models, and will speed up the drug screening process and reduce the late phase attrition rate. The new sensitive insect ex vivo model reduces the risk of saturation of the transporter systems and it improves accuracy of the analytical measurement of the compound concentration in the insect brain.

Hence, the present invention relates to an optimized procedure to increase the sensitivity of existing ex vivo insect models, including that disclosed in international patent application WO2011018446. Thereby the use of low exposure concentrations is enabled. In this regard the inventors have surprisingly found that the brain can be removed from the cuticle without adversely affecting the method disclosed in WO2011018446; instead an increased sensitivity is achieved. It is unexpected that the brain can be disintegrated from the cuticle without making any leaks in the blood-brain barrier. In an additional aspect of the present invention the inventors have found that even the neural lamella can be removed while retaining a reliably model insect. This is also unexpected since removal of the neural lamella would be expected to be detrimental to the functioning of the insect blood-brain barrier.

Accordingly, there is provided a method of conducting blood-brain barrier penetration studies of a chemical compound in an insect, said method comprising the steps:
- optionally anesthetizing the insect;
- fixing the head of the insect;
- dissecting out the brain of the insect head thereby removing the brain from its cuticle;
- optionally removing the neural lamella of the brain;
- treating the brain with a solution of the chemical compound; washing and homogenising the brain;
- determining the concentration of the chemical compound in the homogenised brain material; and
- calculating the penetration of the chemical compound through the blood brain barrier.

In a preferred embodiment of the present invention albumin is added to the solution of the chemical compound to introduce the plasma protein binding and the effect of the plasma protein binding upon the chemical compound's BBB penetration (free vs. protein bound chemical compounds).

Preferable the concentration of the chemical compound is determined by LC/MS. In this respect the determination of the concentration of the chemical compound is performed by homogenizing or ultra sound disintegration (UD) of the dissected brains, and analyzing the concentration of the test agent in the homogenate by liquid chromatography with mass spectrometric detection of the eluted compounds

In order to ensure optimum penetration of the chemical compound the brain is treated with the solution of the chemical compound for a period of 1 min. - 8 hrs. In a particularly preferred embodiment of the present invention the neural lamella of the brain is removed before compound exposure.

The method of the present invention permits the exposure to an insect brain of a test chemical compound solution at a stable concentration during the entire period of exposure. As appears from above the insect model used in the method consists of the isolated brain (including the brain without its neural lamella), which will be treated with different test compound for various times. The permeability of the test chemical compound over the BBB into the brain is determined as the concentration (amount) of the chemical compound measured in the isolated brain and preferably determined by LC/MS. The model is aimed as an early stage test of chemical compounds, in particular drugs, for their ability to cross the BBB at a well defined and constant exposure concentration. BBB permeability is necessary for compounds developed to target CNS related diseases while BBB permeability is considered as a side effect in peripheral acting drugs.

The present invention is applicable for testing chemical compounds' ability to pass the BBB also at low test solution concentration minimizing the risk of saturation of transporter systems. Moreover, the high sensitivity of the invention allows quantification of low BBB permeating compounds at low exposure concentrations. This approach has particular relevance when testing if a given drug may pass the BBB. Thus, the present invention is particularly useful when the chemical compound is a drug, especially a CNS active drug.

The present invention is thus able to provide for the first time rational strategies for screening compounds for neurological indications, as well as generating a simple in vivo system for determining a compound's brain penetration. The present invention is also able to provide a rational screening of compounds in insect models mimicking BBB dysfunction as a consequence of neurological disorders.

Drug discovery is a long and costly process, requiring vast amount of chemical and biological resources. In the present invention the possibilities to use insects as model systems have been thoroughly exploited in order to improve compound selection processes and reduce the costs during the drug discovery phase. Based on recent discoveries the inventors have fully contemplated that insect models of the present invention provide a better foundation than the existing *in vitro* models for selection of compounds to be tested in vertebrates.

The invention is generally applicable to any of a drug discovery programs targeting a variety of diseases and disorders, specifically degenerative disorders, including: Parkinson's Disease, Alzheimer's Disease, Huntington's Disease, Diseases with motor neuron inclusions, Tauopathies, Corticobasal degeneration; Neuropsychiatric disorders, including: Depression Bipolar disease, Schizophrenia, Anxiety, and Aggression. Moreover, the invention is applicable for drug discovery programs targeting peripheral targets where no CNS driven side effect can be tolerated or screening of chemical compounds which effects on CNS functions is unknown.

Thus, the invention is equally applicable to screening for chemical compounds which exert a biological effect that alters an activity or function in the central nervous system, brain or eye, whether normal or subject to a disease or disorder, as to screening for agents which exert a biological effect that is ameliorative of a sign or symptom of a disease or disorder. Moreover, the present invention offers the possibility to test whether or not peripheral acting drugs and toxic agents, such as pesticides, unintentionally penetrate the BBB.

Following identification of a test substance with desired biological activity using a method in accordance with any aspect or embodiment of the present invention the test substance may be formulated into a composition comprising at least one additional component, for example a pharmaceutically acceptable vehicle, carrier or excipient.

### BRIEF DESCRIPTION OF THE DRAWINGS

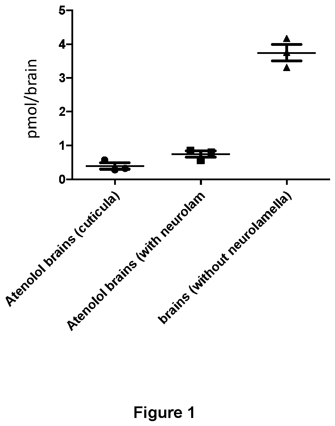

Figure 1 shows the difference sensitivity of three different insect ex vivo methods using a 30uM atenolol solution. It is seen that there is an improved sensitivity of the models when the brains are removed from the cuticle, i.e. the brain concentration of the low permeating compound atenolol is lower when the brain is exposed to atenolol when it is in its cuticula. Moreover, the low variability indicates that removal of the brain from the cuticula surprisingly does not induce any damage to the brain barrier.

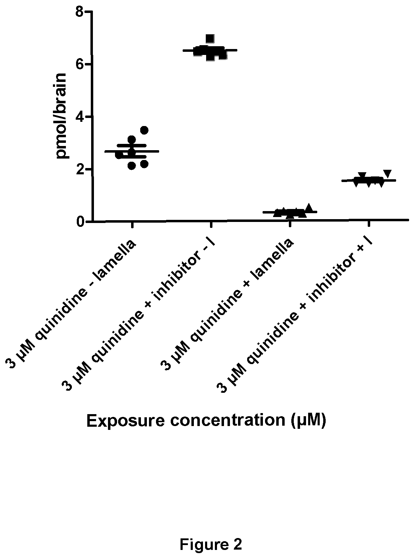

Figure 2 shows that the brain concentration of the Pgp substrate quinidine is higher after co-treatment with the Pgp inhibitor verapamil. This indicates that the Pgp transporter mechanism is fully functional after removal of the brain from the cuticula. Moreover, the figure shows that the Pgp function is retained even after removal of the neural lamella.

### DETAILED DESCRIPTION OF THE INVENTION

The present invention provides new methodology for screening chemical agent's ability to penetrate the BBB also at low exposure concentrations. The invention is generally particular useful for high throughput screening for agents developed in drug discovery programs targeting a variety of diseases and disorders, specifically degenerative disorders, including: Parkinson's Disease, Alzheimer's Disease, Huntington's Disease, Diseases with motor neuron inclusions, Tauopathies, Corticobasal degeneration Neuropsychiatric disorders, including: Depression Bipolar disease, Schizophrenia, Anxiety, and Aggression. Moreover, the invention is applicable for drug discovery programs targeting peripherical targets where no CNS driven side effect can be tolerated. Moreover, the present invention is applicable in the screening of agents developed in drug discovery programs targeting eating disorders and sleep disorders etc.

A drug in accordance with the present invention is defined in its broadest scope as a chemical compound that, when absorbed into the body of a living organism, alters normal bodily function. More specifically, a drug in accordance with the present invention is a chemical compound that may be used in the treatment, cure, prevention, or diagnosis of disease or used to otherwise to enhance physical or mental well-being.

Of particular interest in accordance with the present invention are psychoactive drugs, which are chemical compounds that cross the BBB and acts primarily upon the central nervous system where it alters brain function, resulting in changes in perception, mood, consciousness, cognition and behavior.

The present invention relates to but is not restricted to the use of insects selected from the following orders: (Taxonomy according to: Djurens Värld, Ed B. Hanström; Förlagshuset Norden AB, Malmö, 1964):

| **Order** | **Suborder/family** | **Comment** |
|---|---|---|
| Dictyoptera | Blattoidea | Cockroach |
| | Mantoidea | |
| Orthoptera | Grylloidea | Crickets |
| | Acridoidea | Grasshoppers |
| Cheleutoptera | | Stick insects |
| Lepidoptera | | Moths |
| Hymenoptera | Formicoidea | Ants |
| | Vespoidea | Wasps |
| | Apoidea | Bee like Hymenopterans |
| | Bombinae | Bumble-bees |
| | Apine | Proper bees |
| Odonata | | Dragonflies |
| Diptera | Nematocera | Mosquitos |
| | Brachycera | Flies E.g |
| | | Drosophila |

In particular insect species selected from Blattoidea, Acridoidea, Cheleutoptera, Brachycera and Lepidoptera and most particular Acridoidea (Locusta migratoria and Schistocerca gregaria) are preferred. The invention will also relate to the following orders comprising insect species relevant for the method of the present invention:

| **Order** | **Suborder/family** | **Comment** |
|---|---|---|
| Ephemerida | | Mayflies |
| Plecoptera | | |
| Dermoptera | Forficuloidea | Earwigs |
| Homoptera | Cicadinea | Cicadas |
| | Aphidine | Plant-louse |
| Heteroptera | | Hemipteran |
| Coleoptera | | Beetles |
| Trichoptera | | Caddis fly |

Large insects, such as the migratory locust, Locusta migratoria and the desert locust, Schistocerca gregaria or cockroach where it is feasible to feed and inject drugs and subsequently take hemolymph samples and dissect brain tissues, for analyses, are preferred. The locust has been used to develop screening models to determine BBB penetration of different therapeutic drugs and compare this model with existing literature data from conventional *in vivo* or *in situ* vertebrate studies.

### EXAMPLES

1. In a preferred embodiment the insects are selected from the order Acridoidea and specifically Locusta migratoria and Schistocerca gregaria are used. The insects may be obtained from local suppliers or bred in house. The grasshoppers were reared under crowded conditions at 28° C and a 12:12 dark:light photocycle and fed fresh grass and bran. Before experiments the grasshoppers were fed ecologically cultivated wheat for two weeks. Animals used are adult males (in some experiments females) between two to four weeks after adult emergence. A cut is made through the frontal part of the locust head and the brain is dissected out. The brain is placed in a well of a microtitre plate containing the test solution. After various times of exposure the preparation is washed in saline and the brain is dissected under microscope with fine forceps. The neural lamella surrounding the brain is removed in saline and the brain is then UD in saline, centrifuged and the supernatant frozen until analyses. Drug concentration is analysed by HPLC, LC/MSMS or other methods.
2. In a preferred embodiment the insects are selected from the order Acridoidea and specifically Locusta migratoria and Schistocerca gregaria are used. The insects may be obtained from local suppliers or bred in house. The grasshoppers were reared under crowded conditions at 28° C and a 12:12 dark:light photocycle and fed fresh grass and bran. Before experiments the grasshoppers were fed ecologically cultivated wheat for two weeks. Animals used are adult males (in some experiments females) between two to four weeks after adult emergence. A cut is made through the frontal part of the locust head and the brain is dissected out. The neural lamella is removed from the brain and the brain is placed in a well of a microtitre plate containing the test solution. After various times of exposure the preparation is washed in saline and the brain is dissected under microscope with fine forceps. The brain is then UD in saline, centrifuged and the supernatant frozen until analyses. Drug concentration is analysed by HPLC, LC/MSMS or other methods.
3. In a preferred embodiment the insects are selected from the order Acridoidea and specifically Locusta migratoria and Schistocerca gregaria are used. The insects may be obtained from local suppliers or bred in house. The grasshoppers were reared under crowded conditions at 28° C and a 12:12 dark:light photocycle and fed fresh grass and bran. Before experiments the grasshoppers were fed ecologically cultivated wheat for two weeks. Animals used are adult males (in some experiments females) between two to four weeks after adult emergence. A cut is made through the frontal part of the locust head and the brain is dissected out. The brain is placed in a well of a microtitre plate containing the test solution comprising the substance of interest and 4.2% bovine serum albumin. After various times of exposure the preparation is washed in saline and the brain is dissected under microscope with fine forceps. The neural lamella surrounding the brain is removed in saline and the brain is then UD in saline, centrifuged and the supernatant frozen until analyses. Drug concentration is analysed by HPLC, LC/MSMS or other methods.
4. In a preferred embodiment the insects are selected from the order Acridoidea and specifically Locusta migratoria and Schistocerca gregaria are used. The insects may be obtained from local suppliers or bred in house. The grasshoppers were reared under crowded conditions at 28° C and a 12:12 dark:light photocycle and fed fresh grass and bran. Before experiments the grasshoppers were fed ecologically cultivated wheat for two weeks. Animals used are adult males (in some experiments females) between two to four weeks after adult emergence. A cut is made through the frontal part of the locust head and the brain is dissected out. The neural lamella is removed from the brain and the brain is placed in a well of a microtitre plate containing the test solution comprising the substance of interest and 4.2% bovine serum albumin. After various times of exposure the preparation is washed in saline and the brain is dissected under microscope with fine forceps. The neural lamella surrounding the brain is removed in saline and the brain is then UD in saline, centrifuged and the supernatant frozen until analyses. Drug concentration is analysed by HPLC, LC/MSMS or other methods.

In the following the present invention is exemplified in further detail.

### Example A.

A cut was made through the frontal part of the locust head. Each brain in its cuticle was placed in a well of a microtitre plate containing a 30uM buffered atenolol test solution. After a five minute exposure at 30° C the preparation was washed in ice cold buffer and the brain was dissected under microscope with fine forceps. The neural lamella surrounding the brain was removed in buffer and the brain was then ultra sound disintegrated in buffer, centrifuged for 5 minutes (10000xg at 4°C) and the supernatant analyzed for drug concentration by, LC/MS. The average uptake of atenolol was 0.39 pmol/brain.

### Example B.

A cut was made through the frontal part of the locust head and the brain was dissected out under microscope with fine forceps. Each brain was placed in a well of a microtitre plate containing a 30uM buffered atenolol test solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of atenolol was 0.74 pmol/brain.

### Example C.

A cut was made through the frontal part of the locust head and the brain was dissected out under microscope with fine forceps. The neural lamella was removed from the brain in buffer and each brain was placed in a well of a microtitre plate containing a 30uM buffered atenolol test solution. After a five minute exposure at 30° C the brain was washed in ice cold buffer and then ultra sound disintegrated in buffer, centrifuged for 5 minutes (10000xg at 4°C) and the supernatant analyzed for drug concentration by, LC/MS. The average uptake of atenolol was 3.74 pmol/brain.

### Example D.

A cut was made through the frontal part of the locust head and the brain was dissected out under microscope with fine forceps. Each brain was placed in a well of a microtitre plate containing a 30uM buffered carbamazepine test solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of carbamazepine was 40.3 pmol/brain.

### Example E.

A cut was made through the frontal part of the locust head and the brain was dissected out under microscope with fine forceps. Each brain was placed in a well of a microtitre plate containing a 30uM buffered carbamazepine and 100 uM verapamil test solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of carbamazepine was 40.0 pmol/brain.

### Example F.

A cut was made through the frontal part of the locust head and the brain was dissected out under microscope with fine forceps. Each brain was placed in a well of a microtitre plate containing a 30uM buffered quinidine solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of quinidine was 6.9 pmol/brain.

### Example G.

A cut was made through the frontal part of the locust head and the brain was dissected out under microscope with fine forceps. Each brain was placed in a well of a microtitre plate containing a 30uM buffered quinidine and 100 uM verapamil test solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of quinidine was 21.4 pmol/brain.

### Example H.

A cut was made through the frontal part of the locust head and the brain was dissected out under microscope with fine forceps. Each brain was placed in a well of a microtitre plate containing a 3uM buffered quinidine test solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of quinidine was 0.33 pmol/brain.

### Example I.

A cut was made through the frontal part of the locust head and the brain was dissected out under microscope with fine forceps. Each brain was placed in a well of a microtitre plate containing a 3uM buffered quinidine and 100 uM verapamil test solution. After a five minute exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of quinidine was 1.52 pmol/brain.

### Example J.

A cut was made through the frontal part of the locust head and the brain was dissected out under microscope with fine forceps. The neural lamella was removed from the brain in buffer and each brain was placed in a well of a microtitre plate containing a 3uM buffered quinidine test solution. After a five minute exposure at 30° C the brain was washed in ice cold buffer and then ultra sound disintegrated in buffer, centrifuged for 5 minutes (10000xg at 4°C) and the supernatant analyzed for drug concentration by, LC/MS. The average uptake of quinidine was 2.67 pmol/brain.

### Example K.

A cut was made through the frontal part of the locust head and the brain was dissected out under microscope with fine forceps. The neural lamella was removed from the brain in buffer and each brain was placed in a well of a microtitre plate containing a 3uM buffered quinidine and 100 uM verapamil test solution. After a five minute exposure at 30° C the brain was washed in ice cold buffer and then ultra sound disintegrated in buffer, centrifuged for 5 minutes (10000xg at 4°C) and the supernatant analyzed for drug concentration by, LC/MS. The average uptake of quinidine was 6.50 pmol/brain.

### Example L.

A cut was made through the frontal part of the locust head and the brain was dissected out under microscope with fine forceps. Each brain was placed in a well of a microtitre plate containing a 3uM buffered quinidine test solution. After 45 minutes exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of quinidine was 11.9 pmol/brain.

### Example M.

A cut was made through the frontal part of the locust head and the brain was dissected out under microscope with fine forceps. Each brain was placed in a well of a microtitre plate containing a 3uM buffered quinidine and 100 uM verapamil test solution. After 45 minutes exposure at 30° C the brain was washed in ice cold buffer and the neural lamella surrounding the brain was removed. The brain was then ultra sound disintegrated in buffer, centrifuged for 5 minutes (10000xg at 4°C) and the supernatant analyzed for drug concentration by, LC/MS. The average uptake of quinidine was 22.4 pmol/brain.

### REFERENCES

Abbott, 2005 Dynamics of CNS barriers: evolusion, differtentiation, and modulation. Cell Mol Neurobiol 25: 5-23
Daneman and Barres, 2005 The blood-brain barrier; lessons from moody flies. Cell 123: 9-12
Di, L. and Kerns, E.H. (2003). Profiling drug-like properties in discovery research. Current Opinion in Chemical Biology 7,402-408.
Gaertner, L.S., Murray, C.L., Morris, C.E. (1998). Transepithelial transport of nicotine and vinblastine in isolated malpighian tubules of the tobacco hornworm (Manduca sexta) suggests a P-glycoprotein-like mechanism. The Journal of Experimental Biology 201, 2637-2645.
Garberg, P. et al. (2005). In vitro models for the blood-brain barrier. Toxicology in Vitro 19, 299-334.
Löscher W and Potschka H, 2005 Blood-brain barrier active efflux transporters: ATP binding cassette gene family. NeuroRx 2: 86-98
Mayer F et al., 2009 Evolutionary conservation of vertebrate blood-brain barrier chemoprotective mechanisms in Drosophila. J Neuroscience 29: 3538-3550.
Neuwelt et al., 2008; Strategies to advance translation research into brain barriers. Lancet Neurol 7: 84-96.
Nitta et al., 2003 Size-selective loosening of the blood-brain barrier in Claudine-5-deficient mice. J Cell Biol 161: 653-660.
Pardridge, W.M. (2002). Drug and gene targeting to the brain with molecular Trojan horses. Nature Reviews Drug Discovery 1, 131-139
Pardridge W.M., 2005 Molecular biology of the blood-brain barrier. Mol Biotechnol 30: 57-70.
Summerfield S G et al., 2007. central nervous system drug disposition: The relationship between in situ brain permeability and brain free fraction. J Pharmacol Exp Ther 322: 205-213
Schinkel et al., 1997 Normal viability and altered pharmacokinetics in mice lacking mdrl-type (drug-transporting) P-glycoproteins. PNAS 94: 4028-4033.
Schinkel, A.H. (1999). P-Glycoprotein, a gatekeeper in the blood-brain barrier. Advanced Drug Delivery Reviews 36, 179-194.
Summerfield, S. et al. (2005). Improving the In Vitro Prediction of In Vivo CNS Penetration: Integrating Permeability, Pgp Efflux and Free Fractions in Blood and Brain. Journal of Pharmacology And Experimental Therapeutics*.*
Turksen, K. and Troy, T.-C. (2004). Barriers built on claudins. Journal of Cell Science 117, 2435-2447.
Xia, C.Q., Xiao, G., Liu, N., Pimprale, S., Fox, L., Patten, C.J., Crespi, C.L., Miwa, G., Gan, L.-S. (2006). Comparison of Species Differences of P-Glycoproteins in Beagle Dog, Rhesus Monkey, and Human Using ATPase Activity Assays. Molecular Pharmaceutics 3 (1), 78-86.
Wu V M and Beitel G J , 2004 A junctional problem a apical proportions: epithelial tube-size control by septate junctions in the Drosophila tracheal system. Curr Opin Cell Biol 16: 493-499.
Zlokovic, 2008 The blood-brain barrier in health and chronic neurodegenerative disorders. Neuron 57: 178-201.

## Claims

1. A method of conducting blood-brain barrier penetration studies of a chemical compound in an insect, said method comprising the steps:
• optionally anesthetizing the insect;
• fixing the head of the insect;
• dissecting out the brain of the insect head thereby removing the brain from its cuticle;
• optionally removing the neural lamella of the brain;
• treating the brain with a solution of the chemical compound;
• washing and homogenising or ultra sound disintegrating the brain;
• determining the concentration of the chemical compound in the homogenised brain material; and
• calculating the penetration of the chemical compound through the blood-brain barrier.

2. The method of claim 1, wherein albumin is added to the solution of the chemical compound.

3. The method of claim 1 or 2, wherein the concentration of the chemical compound is determined by LC/MS.

4. The method of any one of the claims 1-3, wherein the brain is treated with the solution of the chemical compound for a period of 1-480 minutes.

5. The method of any one of the claims 1-4, wherein the neural lamella of the brain are removed before treating the brain with a solution of the chemical compound.

6. The method of any one of the preceding claims, wherein the solution of the chemical compound comprises a CNS drug.

## Patentansprüche

1. Verfahren zur Durchführung von Untersuchungen der Blut-Hirn-Schranken-Durchdringung einer chemischen Verbindung in einem Insekt, welches Verfahren die Schritte umfasst:
• wahlweises Anästhesieren des Insektes;
• Fixieren des Kopfes des Insektes;
• Herauspräparieren des Gehirns des Insektenkopfes, wodurch das Gehirn aus seiner Kutikula entfernt wird;
• wahlweises Entfernen der Neurallamellen des Gehirns;
• Behandeln des Gehirns mit einer Lösung der chemischen Verbindung;
• Waschen und Homogenisieren oder Ultraschall-Desintegration des Gehirns;
• Bestimmen der Konzentration der chemischen Verbindung im homogenisierten Gehirnmaterial; und
• Kalkulieren der Durchdringung der chemischen Verbindung durch die Blut-Hirn-Schranke.

2. Verfahren nach Anspruch 1, wobei der Lösung der chemischen Verbindung Albumin zugesetzt wird.

3. Verfahren nach Anspruch 1 oder 2, wobei die Konzentration der chemischen Verbindung von LC/MS bestimmt wird.

4. Verfahren nach einem der Ansprüche 1 bis 3, wobei das Gehirn für einen Zeitraum von 1-480 Minuten mit der Lösung der chemischen Verbindung behandelt wird.

5. Verfahren nach einem der Ansprüche 1 bis 4, wobei vor dem Behandeln des Gehirns mit einer Lösung der chemischen Verbindung die Neurallamellen des Gehirns entfernt werden.

6. Verfahren nach einem der vorhergehenden Ansprüche, wobei die Lösung der chemischen Verbindung ein CNS-Arzneimittel umfasst.

## Revendications

1. Procédé de conduite d'études de pénétration de la barrière hémato-encéphalique d'un composé chimique dans un insecte, ledit procédé comprenant les étapes consistant à:
• anesthésier éventuellement l'insecte;
• fixer la tête de l'insecte;
• disséquer le cerveau hors de la tête de l'insecte de manière à enlever le cerveau de sa cuticule;
• éliminer éventuellement les lamelles de neurones du cerveau;
• traiter le cerveau avec une solution du composé chimique;
• effectuer un lavage et une homogénéisation ou une désintégration ultrasonore du cerveau;
• déterminer la concentration du composé chimique dans la matière homogénéisée du cerveau; et
• calculer la pénétration du composé chimique à travers la barrière hémato-encéphalique.

2. Procédé selon la revendication 1, dans lequel l'albumine est ajoutée à la solution du composé chimique.

3. Procédé selon la revendication 1 ou 2, dans lequel la concentration du composé chimique est déterminée par CL/SM.

4. Procédé selon l'une quelconque des revendications 1 à 3, dans lequel le cerveau est traité avec une solution du composé chimique pendant une durée comprise entre 1 et 480 minutes.

5. Procédé selon l'une quelconque des revendications 1 à 4, dans lequel les lamelles de neurones du cerveau sont enlevées avant le traitement du cerveau avec une solution du composé chimique.

6. Procédé selon l'une quelconque des revendications précédentes, dans lequel la solution du composé chimique comprend un médicament du SNC.
